Europäisches Patentamt

European Patent Office

Office européen des brevets

(19)

(11) **EP 0 809 485 B1**

(12) **FASCICULE DE BREVET EUROPEEN**

(45) Date de publication et mention
de la délivrance du brevet:
**07.11.2001 Bulletin 2001/45**

(51) Int Cl.[7]: **A61K 7/48**, A61K 7/00,
A61K 7/06, C11D 3/20,
C11D 3/00

(21) Numéro de dépôt: **96939251.3**

(22) Date de dépôt: **06.12.1996**

(86) Numéro de dépôt international:
**PCT/IB96/01370**

(87) Numéro de publication internationale:
**WO 97/22332 (26.06.1997 Gazette 1997/27)**

(54) **UTILISATION DU 4-TERT-BUTYL-1-CYCLOHEXANOL EN TANT QU'ANTIOXYDANT**

VERWENDUNG VON 4-TERT-BUTYL-1-CYCLOHEXANOL ALS ANTIOXIDANT

USE OF 4-TERT-BUTYL-1-CYCLOHEXANOL AS AN ANTIOXIDANT

(84) Etats contractants désignés:
**CH DE ES FR GB IT LI NL**

(30) Priorité: **19.12.1995 CH 357995**

(43) Date de publication de la demande:
**03.12.1997 Bulletin 1997/49**

(73) Titulaire: **FIRMENICH SA**
**1211 Genève 8 (CH)**

(72) Inventeur: **HOLZNER, Günter**
**CH-1212 Grand-Lancy (CH)**

(74) Mandataire: **Salvaterra-Garcia, Maria de Lurdes**
**Firmenich SA**
**Département des Brevets**
**Case Postale 239**
**1211 Genève 8 (CH)**

(56) Documents cités:
**EP-A- 0 427 965**

**Description**

La présente invention a trait au domaine de la parfumerie et de la cosmétique. Elle concerne, plus particulièrement, l'utilisation du 4-tert-butyl-1-cyclohexanol en tant qu'agent antioxydant.

Le 4-tert-butyl-l-cyclohexanol est un ingrédient parfumant connu, mais dont l'usage n'est pas très courant, en raison de sa note camphrée pas trop élégante. Il développe en effet une odeur de type boisé-patchouli, camphrée. On sait également que ce composé se montre très stable même dans des milieux agressifs caractéristiques notamment des savons, détergents ou encore des produits de blanchissage. Cependant, à notre connaissance il n'a jamais été suggéré d'utiliser ce composé en tant que stabilisant contre l'oxydation dans des produits de nettoyage ou cosmétiques.

**Description de l'invention**

Or, nous venons maintenant de découvrir que le 4-tert-butyl-1-cyclohexanol se révèle très efficace en tant qu'agent antioxydant, notamment lorsqu'il est utilisé dans des milieux détergents et cosmétiques contenant des matières grasses qui s'oxydent facilement à l'air et/ou à la lumière et dont les produits d'oxydation développent des odeurs désagréables, notamment de type rance.

On sait, par exemple, que certains savons développent des odeurs rances, piquantes et fruitées après seulement quelques semaines de stockage à l'air. Or, nous avons constaté que ces mauvaises odeurs pouvaient être empêchées ou du moins évitées pendant une période de stockage beaucoup plus longue, si on leur ajoutait du 4-tert-butyl-1-cyclohexanol.

Des effets similaires ont été observés avec des shampoings ou encore des gels de douche ou bain.

D'une façon générale, ce composé se révèle un stabilisant efficace contre l'oxydation dans tous les produits cosmétiques ou de nettoyage de la peau ou des cheveux contenant des matières pouvant s'oxyder à l'air et/ou à la lumière et développer ainsi des mauvaises odeurs. On peut citer à cet effet, en plus des produits de consommation mentionnés plus haut, les crèmes et laits cosmétiques.

Il a aussi été constaté que ce composé pouvait être utile en tant qu'agent antioxydant dans d'autres produits de nettoyage en général, en particulier dans les détergents à usages multiples, notamment pour le nettoyage de surfaces dures, de la vaisselle, etc ("all-purpose cleaners"), lorsqu'ils contiennent des matières grasses susceptibles d'oxydation, à l'air et/ou à la lumière.

Pour produire les effets désirés selon l'invention, le 4-tert-butyl-1-cyclohexanol peut être ajouté à la base de type savon, shampoing ou autre, soit seul, soit en mélange avec d'autres ingrédients d'usage courant en parfumerie, typiquement les ingrédients parfumants, solvents ou adjuvants usuels. Une description plus détaillée de ces ingrédients est ici superflue. Leur nature dépendra notamment de l'effet parfumant désiré, ainsi que du type de produit que l'on désire parfumer, et l'homme de l'art est à même de les choisir sur la base de ses connaissances générales et en s'inspirant notamment d'oeuvres de référence telles que l'ouvrage de S. Arctander, Perfume and Flavor Chemicals, Montclair, N.J. (1969), ou les versions plus récentes de cet ouvrage, parmi d'autres.

Les concentrations dans lesquelles le composé susmentionné peut être ajouté aux produits divers pour déployer son effet antioxydant varient dans une gamme de valeurs étendue. Des effets bénéfiques peuvent être déjà constatés à des concentrations de l'ordre de 0,005 ou 0,01% en poids, par rapport au poids du produit fini, à savoir le savon, shampoing, gel de douche ou autre. On préfère, cependant, utiliser ce composé dans des concentrations égales ou supérieures à 0,05% en poids, par rapport au poids du produit fini. Préférentiellement, ces produits contiendront à raison de 0,05 à 0,5% en poids de 4-tert-butyl-1-cyclohexanol.

Comme il ressort des exemples présentés plus loin, le 4-tert-butyl-1-cyclohexanol se révèle être un antioxydant plus efficace que d'autres agents couramment utilisés à cet effet tels que le BHT (2,6-di-tert-butyl-p-crésol ; origine : Bayer), l'$\alpha$- ou le $\gamma$-tocophérol, les acides ascorbique, citrique ou tartrique, leurs sels ou esters, ou encore des mélanges de deux ou plusieurs de ces produits. Bien entendu, on peut également utiliser le 4-tert-butyl-1-cyclohexanol en mélange avec ces antioxydants. Nous avons observé en particulier que d'excellents résultats pouvaient être obtenus lorsque le 4-tert-butyl-1-cyclohexanol était utilisé en combinaison avec les tocophérols ($\alpha$- ou $\gamma$), l'acide citrique, l'acide ascorbique ou l'acide tartrique, ou encore leurs esters.

Comme il a été cité auparavant, le 4-tert-butyl-1-cyclohexanol peut être utilisé en tant qu'antioxydant dans des compositions parfumantes, cosmétiques ou de nettoyage de la peau ou des cheveux, ou encore dans des compositions détergentes à usages multiples, et l'invention se rapporte aussi à ces compositions.

La nature des compositions de base auxquelles on ajoute ce composé, peut-être quelconque, toutes les bases de type savon, cosmétiques ou détergentes d'usage courant dans l'industrie correspondante ou connues dans l'art pouvant servir à préparer les compositions de l'invention dans lesquelles le 4-tert-butyl-1-cyclohexanol est incorporé en tant qu'agent anti-oxydant. Les exemples de ces compositions de base sont cités à titre illustratif mais il est clair que d'autres bases du même type d'usage courant contenant des matières oxydables, peuvent être utilisées à la place de celles exemplifiées.

En particulier, les compositions cosmétiques ou de nettoyage de la peau ou des cheveux qui contiennent 0,05 à 0,5% en poids de ce composé, par rapport au poids de la composition, sont préférées selon l'invention. Les savons, les shampoings ou encore les gels de bain ou douche sont des modes d'exécution de ces compositions selon l'invention, à l'instar des crèmes et laits hydratants, de beauté, ou autres.

Par ailleurs, il convient de noter que l'on sait que dans certains milieux, propices à l'hydrolyse, l'addition de l'acétate de 4-tert-butyl-1-cyclohexyle peut être équivalente à celle du 4-tert-butyl-1-cyclohexanol, ledit acétate pouvant s'hydrolyser partiellement ou totalement pour former cet alcool correspondant. Il est clair donc que l'invention concerne également les compositions et produits du type susmentionné, auxquels on ajouterait, ou qui contiendraient, ledit acétate dont les conditions de pH sont susceptibles de provoquer la formation de l'alcool correspondant, en quantités suffisantes pout que ce dernier déploie son activité antioxydante dans la composition ou produit.

Lorsqu'utilisé dans des compositions parfumantes, lesquelles peuvent être ajoutées aux produits susmentionnés pour leur impartir une odeur déterminée, le 4-tert-butyl-1-cyclohexanol pourra constituer de 5 à 50% du poids de la composition parfumante.

L'invention sera maintenant décrite plus en détail à l'aide des exemples suivants, dans lesquels les températures sont indiquées en degrés centigrades.

## Manières de réaliser l'invention

Exemple 1

Stabilisation d'un savon de type semi-synthétique

On a préparé une base de savon en mélangeant les ingrédients suivants, selon des méthodes courantes.

| Ingrédients | % en poids |
|---|---|
| Cocoyliséthionate de sodium [1] | 51,0 |
| Dodecylbenzènesulfonate de sodium [2] | 2,0 |
| Iséthionate de sodium [3] | 5,0 |
| Acide stéarique | 25,0 |
| Sel de sodium d'acides gras de $C_{12}$ à $C_{18}$ | 17,0 |
| Total | 100,0 |

1) origine : PPG Industries, USA

2) origine : Chem. Werke Hüls, Allemagne

3) origine : Witco Chem. Comp., USA

A cette composition de base on a ajouté les ingrédients cités au tableau ci-après, dans les quantités indiquées, pour préparer des échantillons stabilisés de base de savon.

TABLEAU I

| Ingrédients | Echantillon % en poids, par rapport à la base | | | | |
|---|---|---|---|---|---|
| | A | B | C | D | E |
| Tenox®[1] GT II | - | 0,07 | - | 0,05 | - |
| Irganox®[2] PS-800 | 0,05 | - | 0,05 | 0,05 | - |
| Acide citrique* | 0,20 | 0,20 | 0,20 | 0,20 | 0,20 |
| BHT | 0,07 | - | 0,07 | - | - |
| Turpinal®[3] SL | - | - | 0,10 | - | - |
| 4-tert-butyl-1-cyclohexanol | - | - | - | 0,1 | 0,2 |

* à 50% dans l'eau

1) α-tocophérol ; origine : Eastman Chemicals

2) dilaurylthiodipropanoate ; origine : Ciba-Geigy AG

3) acide étidronique; origine : Henkel

Des savons ont été frappés de façon conventionnelle à l'aide de ces bases stabilisées. Les savons A à E ainsi obtenus ont été stockés dans des récipients en verre pendant un mois, à 40° et ensuite évalués à l'aveugle par un panel d'experts parfumeurs. Le panel a jugé que les savons A à C avaient une odeur grasse, de beurre et rance que l'on ne retrouvait ni dans le savon D, ni dans le savon E, lesquels exhalaient en plus une fragrance légèrement florale.

Exemple 2

Stabilisation de savons solides

On a préparé un mélange stabilisateur à l'aide de 1 g de 4-tert-butyl-1-cyclohexanol, 0,5 g de Tenox® GT II et 0,5 g d'Irganox® PS-800.

A l'aide de la composition de base décrite dans l'exemple 1, on a ensuite préparé des bases de savon parfumées, en ajoutant les ingrédients indiqués ci-après, dans les quantités citées :

TABLEAU II

| Ingrédients | Echantillon % en poids, basé sur le poids de la composition de base | | | | |
|---|---|---|---|---|---|
| | A | B | C | D | E |
| Hédione®[1] | 0,05 | 0,05 | 0,05 | 0,05 | 0,05 |
| Essence de bois de rose | 0,1 | - | - | - | - |
| Essence de bergamote | - | 0,1 | - | - | - |
| Essence de benjoin * | - | - | 0,1 | - | - |
| Essence de cèdre | - | - | - | 0,1 | - |
| Essence de géranium | - | - | - | - | 0,1 |

* à 20% dans le dipropylèneglycol

1) dihydrojasmonate de méthyle ; origine : Firmenich SA, Genève, Suisse

Des savons A à E ont été frappés avec ces bases de savons. Parallèlement, des savons A' à E' ont été obtenus à partir des bases A à E, auxquelles on avait ajouté en plus à raison de 0,3% du mélange stabilisateur cité plus haut.

Les dix savons ont été stockés à 40° pendant un mois et ensuite évalués à l'aveugle deux à deux, c'est-à-dire A et A', B et B', etc, par un panel d'experts.

Le résultat de ces évaluations a montré que tous les savons A' à E' avaient une bonne odeur sans aucune note grasse, rance, alors que les savons A à E développaient des fragrances où des notes rances désagréables pouvaient être clairement détectées.

Exemple 3

Stabilisation de savons solides

On a préparé une base de savon standard en mélangeant, de façon connue en soi les ingrédients suivants :

| Ingrédients | % en poids |
|---|---|
| Sels de sodium d'acides gras de suif de boeuf | 60,0 |
| Sels de sodium d'acides gras d'huile de coco | 26,0 |
| Eau | 14,0 |
| Total | 100,0 |

A cette base de savon on a alors ajouté à raison de 1,5 à 2% en poids de l'une des compositions parfumantes A ou B contenant les ingrédients suivants, pour préparer des bases de savon parfumées :

| Ingrédients | Composition A | Composition B |
|---|---|---|
| | (Parties en poids) | |
| Phénéthylol | 3500 | 3500 |
| Citronellol | 2000 | 2000 |
| Iralia®[1] | 300 | 300 |
| Géraniol | 600 | 600 |
| Diphényl éther | 200 | 200 |
| Acétate de benzyle | 800 | 800 |
| Acétate de phényléthyle | 400 | 400 |
| 3-Méthyl-5-phényl-1-pentanol [2] | 400 | 400 |
| Dorinia SA [3] | 100 | 100 |
| Vert de lilas [4] | 200 | 200 |
| 15-Pentadec-(11,12)-énolide [5] à 50% * | 50 | 50 |
| 4-tert-butyl-1-cyclohexanol | -- | 1000 |
| Total | 9000 | 10000 |

* dans le dipropylèneglycol

1) méthylionone ; origine : Firmenich SA, Genève, Suisse

2) origine : Firmenich SA, Genève, Suisse

3) origine : Firmenich SA, Genève, Suisse

4) (2,2-diméthoxyéthyl)benzène ; origine : Givaudan-Roure, Vernier, Suisse

5) origine : Firmenich SA, Genève, Suisse

Des savons A et B ont été frappés de façon courante à partir des bases de savons parfumées à l'aide des compositions A, respectivement B. Après un mois de stockage à 40°, les deux savons ont été évalués à l'aveugle par un panel d'experts parfumeurs. De l'avis de ces derniers, le savon B dégageait une odeur de type floral, rose, vert, musqué, alors que le savon A présentait des notes rances, grasses clairement perceptibles, qui rendaient son odeur nettement moins plaisante que celle du savon B.

Exemple 4

Préparation d'un shampoing liquide

On a préparé un shampoing liquide pour le nettoyage du corps et des cheveux en mélangeant, selon les techniques usuelles, les ingrédients suivants :

| Ingrédients | % en poids |
|---|---|
| Texapon®[1]N28 | 25,0 |
| Medialan®[2] LD | 7,0 |
| Eau déminéralisée | 64,4 |
| NaCl | 1,5 |
| Acide citrique | 0,5 |
| Kathon®[3] CG | 0,1 |
| Comperland®[4] KD | 1.5 |
| Total | 100,0 |

1) éthersulfate de sodium ; origine : Henkel

2) sarcosinate de lauryle ; origine : Hoechst

3) conservateur ; origine : Röhrn & Haas

4) diéthanolamide de l'acide de coco ; origine : Henkel

A ce shampoing de base non parfumé on a ajouté 0,1% en poids de 4-tert-butyl-1-cyclohexanol, 0,05% en poids de Tenox® GII, 0,05% en poids d'Irganox® PS-800 et 0,1% en poids d'acide citrique pour obtenir un shampoing stabilisé.

On a rempli des récipients en plastique avec ce shampoing stabilisé et avec le shampoing de base.

Après un mois de stockage à 40°, les deux shampoings ont été évalués à l'aveugle par un panel d'experts. Ces derniers ont indiqué une préférence marquée pour le shampoing stabilisé lequel ne présentait aucune trace de notes grasses, alors que le shampoing de base avait une odeur âcre et grasse bien marquée.

Exemple 5

Test de stabilité

Afin de tester l'effet antioxydant du 4-tert-butyl-1-cyclohexanol dans des compositions parfumantes susceptibles de s'oxyder à l'air, on a ajouté à un mélange de terpènes d'orange (contenant 95% en poids de limonène), 0,1% en poids du cyclohexanol susmentionné, 0,05% en poids d'Irganox® PS-800 et 0,05% en poids de Tenox® GT II.

On a ensuite soumis le mélange de terpènes seul, et le mélange de terpènes avec les stabilisants susmentionnés à un test du type Rancimat (voir par exemple, M. W. Làubli, Lebensmittelchemie 48, page 134). Pour ce faire, on a chauffé les deux mélanges à 110°, sous atmosphère d'air, en mesurant leur conductivité. On a ainsi observé que la conductivité du mélange de terpènes indiquait l'oxydation de celui-ci après 2h de chauffage, alors que le mélange contenant les stabilisants ne commençait à s'oxyder qu'après 5,5 h de chauffage à l'air.

Exemple 6

Stabilisation d'un produit de nettoyage à usages multiples

On a préparé un produit de nettoyage de surfaces ou à usages multiples ("all-purpose cleaner") par mélange des ingrédients suivants, selon les techniques courantes dans l'art.

| Ingrédients | % en poids |
|---|---|
| Eau | 74,7 |
| Carbonate de sodium | 3,0 |
| Citrate de sodium | 2,0 |
| Cumolsulphonate de sodium [1] | 7,0 |
| Marlon®[1][2] A 375 | 10,0 |
| Tergitol ® [3] 15-S-9 | 3,0 |
| Parfum [4] | 0,3 |
| Total | 100,0 |

1) origine : Hüls AG, Allemagne

2) sulfonate de dodecylbenzène sodium ; origine : Hüls AG, Allemagne

3) alcool secondaire éthoxylé $C_{11}$-$C_{15}$ ; origine : Union Carbide, USA

4) Blue Water n° 114 338 ; origine : Firmenich SA, Suisse

A cette composition de base parfumée, on a encore ajouté 0,1% en poids d'huile d'amande douce, pour obtenir une composition de nettoyage tous usages de base. Avec cette dernière, on a alors préparé une composition détergente selon l'invention, par addition de 0,2% en poids de 4-tert-butyl-1-cyclohexanol. La composition de base et cette nouvelle composition ont ensuite été stockées dans des flacons en plastique pendant 8 h, sous lumière ultraviolette. A la fin de ce stockage, ils ont été évalués à l'aveugle par un panel d'experts, lesquels ont indiqué que le produit contenant le 4-tert-butyl-1-cyclohexanol ne présentait aucune trace de notes grasses, alors que le détergent de base avait une odeur d'huile d'amande rance.

**Revendications**

1. Utilisation du 4-tert-butyl-1-cyclohexanol en tant qu'agent antioxydant.

2. Utilisation selon la revendication 1, pour la préparation d'une composition parfumante, cosmétique, de nettoyage de la peau ou des cheveux ou de nettoyage de surfaces.

3. Utilisation selon la revendication 1 ou 2, **caractérisée en ce que** le 4-tert-butyl-1-cyclohexanol est en combinaison avec l'α- ou γ-tocophérol, l'acide ascorbique, l'acide citrique, l'acide tartrique ou un ester de l'un de ces acides.

**4.** Composition parfumante **caractérisée en ce qu'**elle contient, en tant qu'agent antioxydant, le 4-tert-butyl-1-cyclohexanol, dans une concentration comprise entre 5 et 50% en poids, basé sur le poids de la composition.

**5.** Composition cosmétique, de nettoyage de la peau ou des cheveux ou de nettoyage de surfaces, contenant, en tant qu'agent antioxydant, le 4-tert-butyl-1-cyclohexanol, dans une concentration d'au moins 0,05% en poids, par rapport au poids de la composition.

**6.** Composition selon la revendication 5, **caractérisée en ce que** la concentration de 4-tert-butyl-1-cyclohexanol est comprise entre 0,05% et 0,5% en poids, du poids de la composition.

**7.** Composition selon l'une des revendications 4 à 6, **caractérisée en ce que** le 4-tert-butyl-1-cyclohexanol est en combinaison avec l'α- ou γ-tocophérol, l'acide ascorbique, l'acide citrique, l'acide tartrique ou un ester de l'un de ces acides.

**8.** Savon, gel de douche ou bain ou shampoing contenant à raison de 0,05 à 0,5% en poids de 4-tert-butyl-1-cyclohexanol.

**9.** Savon, gel de douche ou bain ou shampoing selon la revendication 8, **caractérisé en ce que** le 4-tert-butyl-1-cyclohexanol est en combinaison avec l'α- ou γ-tocophérol, l'acide ascorbique, l'acide citrique, l'acide tartrique ou un ester de l'un de ces acides.

**10.** Produit de nettoyage de surfaces, **caractérisé en ce qu'**il contient à raison de 0,05 à 0,5% en poids de 4-tert-butyl-1-cyclohexanol.

**Patentansprüche**

**1.** Verwendung von 4-*tert*-Butyl-1-cyclohexanol als Antioxidans.

**2.** Verwendung nach Anspruch 1 für die Herstellung einer Duftzusammensetzung, kosmetischen Zusammensetzung, Zusammensetzung zum Reinigen der Haut oder der Haare oder zum Reinigen von Oberflächen.

**3.** Verwendung nach Anspruch 1 oder 2, **dadurch gekennzeichnet, daß** das 4-*tert*-Butyl-1-cyclohexanol in Kombination mit α- oder γ-Tocopherol, Ascorbinsäure, Zitronensäure, Weinsäure oder einem Ester einer dieser Säuren vorliegt.

**4.** Duftzusammensetzung, **dadurch gekennzeichnet, daß** sie als Antioxidans 4-*tert*-Butyl-1-cyclohexanol in einer Konzentration enthält, die zwischen 5 und 50 Gew.-% bezogen auf das Gewicht der Zusammensetzung beträgt.

**5.** Kosmetische Zusammensetzung, Zusammensetzung zum Reinigen der Haut oder der Haare oder zum Reinigen von Oberflächen, welche als Antioxidans 4-*tert*-Butyl-1-cyclohexanol in einer Konzentration von mindestens 0,05 Gew.-% bezogen auf das Gewicht der Zusammensetzung enthält.

**6.** Zusammensetzung nach Anspruch 5, **dadurch gekennzeichnet, daß** die Konzentration von 4-*tert*-Butyl-1-cyclohexanol zwischen 0,05 Gew.-% und 0,5 Gew.-% des Gewichts der Zusammensetzung beträgt.

**7.** Zusammensetzung nach einem der Ansprüche 4 bis 6, **dadurch gekennzeichnet, daß** das 4-*tert*-Butyl-1-cyclohexanol in Kombination mit α- oder γ-Tocopherol, Ascorbinsäure, Zitronensäure, Weinsäure oder einem Ester einer dieser Säuren vorliegt.

**8.** Seife, Dusch- oder Badegel oder Shampoo, welche(s) 4-*tert*-Butyl-1-cyclohexanol in einer Menge von 0,05 bis 0,5 Gew.-% enthält.

**9.** Seife, Dusch- oder Badegel oder Shampoo nach Anspruch 8, **dadurch gekennzeichnet, daß** das 4-*tert*-Butyl-1-cyclohexanol in Kombination mit α- oder γ-Tocopherol, Ascorbinsäure, Zitronensäure, Weinsäure oder einem Ester einer dieser Säuren vorliegt.

**10.** Produkt zum Reinigen von Oberflächen, **dadurch gekennzeichnet, daß** es 4-*tert*-Butyl-1-cyclohexanol in einer Menge von 0,05 bis 0,5 Gew.-% enthält.

**Claims**

1. Use of 4-tert-butyl-1-cyclohexanol as an antioxidant.

2. Use according to claim 1, for the preparation of a perfuming or cosmetic composition, of a skin or hair cleaning composition, or of a composition for cleaning surfaces.

3. Use according to claim 1 or 2, **characterized in that** 4-tert-butyl-1-cyclohexanol is in combination with $\alpha$- or $\gamma$-tocopherol, ascorbic acid, citric acid, tartric acid or an ester of one of these acids.

4. Perfuming composition **characterized in that** it comprises, as antioxidant 4-tert-butyl-1-cyclohexanol, in a concentration of between 5 and 50% by weight, based on the weight of the composition.

5. Cosmetic composition or cleaning composition for skin or hair, or for cleaning surfaces, comprising as antioxidant 4-tert-butyl-1-cyclohexanol, in a concentration of at least 0.05% by weight, relative to the weight of the composition.

6. Composition according to claim 5, **characterized in that** the concentration of 4-tert-butyl-1-cyclohexanol is of between 0.05% and 0.5% by weight, relative to the weight of the composition.

7. Composition according to any one of claims 4 to 6, **characterized in that** 4-tert-butyl-1-cyclohexanol is in combination with $\alpha$- or $\gamma$-tocopherol, ascorbic acid, citric acid, tartric acid or an ester of one of these acids.

8. Soap, bath or shower gel or shampoo comprising about 0.05 to 0.5% by weight of 4-tert-butyl-1-cyclohexanol.

9. Soap, bath or shower gel or shampoo according to claim 8, **characterized in that** 4-tert-butyl-1-cyclohexanol is in combination with $\alpha$- or $\gamma$-tocopherol, ascorbic acid, citric acid, tartric acid or an ester of one of these acids.

10. Surface cleaner, comprising about 0.05 to 0.5% by weight of 4-tert-butyl- 1 -cyclohexanol.